# EUROPEAN PATENT SPECIFICATION

(11) **EP 3 946 011 B1**
(45) Date of publication and mention of the grant of the patent: **07.02.2024**
(21) Application number: 20721302.6
(22) Date of filing: 01.04.2020
(51) Int. Cl.: A61B 5/00, A61B 5/103, A61B 5/107

(54) **SYSTEM FOR THE DETERMINATION OF THE SURFACE AND MECHANICAL CHARACTERISTICS OF FILAMENTARY STRUCTURES, IN PARTICULAR SKIN APPENDAGES, STRUCTURES ASSOCIATED THEREWITH, NATURAL OR SYNTHETIC FIBERS AND THEIR AGGREGATES**
SYSTEM ZUR BESTIMMUNG DER OBERFLÄCHE UND DER MECHANISCHEN EIGENSCHAFTEN VON FADENFÖRMIGEN STRUKTUREN, INSBESONDERE HAUTANHÄNGSELN, DAMIT VERBUNDENE STRUKTUREN, NATÜRLICHE ODER SYNTHETISCHE FASERN UND DEREN AGGREGATE
SYSTÈME DE DÉTERMINATION DES CARACTÉRISTIQUES DE SURFACE ET MÉCANIQUES DE STRUCTURES FILAMENTEUSES, EN PARTICULIER DES PHANÈRES, STRUCTURES ASSOCIÉES, FIBRES NATURELLES OU SYNTHÉTIQUES ET LEURS AGRÉGATS

(30) Priority: 01.04.2019 IT 201900004861
(43) Date of publication of application: 09.02.2022
(73) Proprietor: Universita' Degli Studi Di Pavia, 27100 Pavia (IT)
(72) Inventor: PERUGINI, Paola, 27049 Stradella (Pavia) (IT); MUSITELLI, Giorgio, 27058 Voghera (Pavia) (IT)
(74) Representative: Deambrogi, Edgardo
(86) International application number: PCT/IB2020/053079
(87) International publication number: WO 2020/202018

(56) References cited:
- WO-A1-95/29396
- WO-A1-2016/137908
- WO-A2-2008/072151
- DE-A1-102004 037 566
- US-A- 5 167 150
- US-A1- 2012 320 191

## Description

The present invention relates to the analysis of filamentary structures, in particular filamentary structures such as vital tissues, natural or synthetic fibers.

More specifically, the present invention relates to the quantitative-quantitative study of the surface characteristics, mechanical properties, and, indirectly, structural properties of filaments, in particular vital or reconstituted tissues, in particular skin appendages, more particularly hair, eyelashes, or structures associated therewith.

Skin appendages are structures which have a close functional link and a common embryological origin with the skin. Amongst these, the hair and eyelashes are epidermal formations placed respectively on the head and on the end of the eyelids, which, from a physiological point of view, provide a function mainly of protection.

The hair and eyelashes are constituted prevalently by keratin, a fibrous scleroprotein which is responsible for most of the mechanical properties of such structures.

The optical properties of the hair (or eyelash) in itself, and of hairstyles as a whole, depend to a large extent both on the outermost and surface part of the hair, i.e. the cuticle, and on the more or less distinctive presence of pigments (such as, for example, melanin and/or natural and/or synthetic pigments) situated in the various portions of the hair (cuticle, cortex and medulla). Both the mechanical and optical properties can be more or less distinctly affected and modified as a result of mechanical, physical or chemical treatments, or as a result of the application and depositing of various substances. Many of the treatments to which these skin appendages are subjected are closely associated with the lifestyle of modem Western culture, the roots themselves of which lifestyle is based in an extremely old and well-established tradition. By way of example, it is sufficient to mention the processes of dyeing, and of temporary or permanent modification of the hairstyle which constitute some of the most important habits associated with modern lifestyle.

Products for the health and care of the hair which comprise, inter alia, detergents, conditioners, fixatives, protection agents, etc., together with those for the make-up and treatment of the eyelashes, are playing a strongly increasing part in the cosmetics and pharmaceuticals market.

In parallel with the growth of the market for cosmetics products, the effects claimed by such products have also increased. For this purpose, at European level, (CE) Regulation 1223/2009 has been introduced, and has been in force since July 2013. Amongst other things, this Regulation makes it compulsory to verify the truthfulness of properties claimed which are not strictly correlated with the nature itself of the cosmetic product. For this purpose, it is essential to have and validate instrumental methods which can verify these effects. In particular, in order to verify the effectiveness and performance levels of a cosmetic formulation, it is mandatory to develop adequate experimental designs, carrying out evaluations which are preferably "in vivo," in other words in real conditions of use, which can take place for quite different periods of time, varying from a few minutes to months, in relation to the type of product and the functionalities to be ascertained.

In view of the enormous interest relating to the study of hair, to the formulative development in this context and to the corresponding evaluation of effectiveness, there are numerous approaches, and consequently numerous technologies which have been developed and adapted for the study of the surface characteristics of the hair. In the first instance, reference is made here to the surface characteristics, since these are the focus of attention of most of the products and treatments for eyelashes and hair, and consequently the properties claimed by the corresponding advertising campaigns.

In general it can be said that, in order to characterize the surface properties of the hair, recourse is usually made to two options:
- a first, indirect option which uses the study of some mechanical properties of a sample, for example of hair, which are used to deduce other properties therefrom, on the basis of any variation thereof, with deduction and/or reconstruction of the effect of a treatment;
- a second, direct option, which is based on the direct study of suitably acquired images which, after reprocessing, can provide information on the optical properties of the sample analyzed. These images typically contain and analyze profiles of reflection and diffusion relative to a source of light which irradiates the sample.

The first method of approach includes a vast range of equipment, which in most cases can determine the friction which a probe of varied geometry encounters when passing through the sample. One of the most common and widely used examples of this technique is constituted by the combability test. This test consists of passing a probe, commonly shaped as a comb or brush, through a lock of hair, and measuring the friction thereof which the probe encounters. An exclusively mechanical example of this approach is found in patent US 3,459,197 entitled "Comb-mounted hair analysis gauge" in which there is measurement of the deflection of a comb which is deformed because of the friction which it encounters during its use. This deformation is recorded on a graduated scale.

US 4,167,869 entitled "Apparatus for measuring hair grooming force" records the same parameter, i.e. the friction encountered by a comb which is applied to a lock of hair, basing the reading of the datum on the modification of the probe resistance values associated with the comb. The variation of these resistance values is translated into an electronic signal which permits continuous recording of the friction value concerned.

Although based on the same structural plan, some technological solutions record and amplify for example the noise or the vibrations produced by the probe whilst it is being moved on the sample. Examples of such technological solutions are described in the Japanese patent application JP 2004 159830 entitled "Hair evaluation system" or in the Japanese patent application JP 2009 201812 entitled "Sensor for measuring hair characteristic and evaluation apparatus and evaluation method using the same."

All the examples referred to hitherto can be used both on samples prepared ex vivo and on locks of hair in vivo.

However, structures exist which can be considered as accessories of common tensiometers, by means of the use of which it is possible to carry out tests ex vivo with quantification, as in some cases previously cited, of the resistance encountered by a probe substantially in the shape of a comb, whilst it is slid on a sample of hairs. The main advantage of this technique consists of the possibility of implementing a regular movement of the probe. An example of an accessory of this type is the product known as a "hair combing rig" made by the company Stable Micro System.

The second method of approach includes a series of equipment which is extremely sophisticated, and is often unable to operate on samples in vivo, which equipment further requires laborious and costly preparation of the sample in order to be able to carry out the analysis, as well as needing image capturing apparatuses which are even more sophisticated and costly. Examples of this approach are various goniophotometry techniques often associated with DSLR (Digital Single-Lens Reflex) cameras and with polarized sources of light. In some cases, analysis in vivo is possible, as for example in the system identified as Samba Hair System, produced by the US company BOSSA NOVA TECHNOLOGIES.

The subject of US 2012/320191 is a method and a device for analyzing hair fibers by means of the positioning of the fibers on an image sensor which receives light from a source and evaluates the images of the fibers by means of a processor, correlating in succession the analysis values generated by the processor with predetermined descriptors of the properties of the hair. The invention according to US 2012/320191 A1 can potentially also analyze in vivo a suitably irradiated and photographed sample of hairs. However, this equipment, in addition to capturing and analyzing images generated exclusively by the radiation transmitted by the irradiated sample, is not at all suitable for the study of samples constituted by numerous elements such as the hairs of an entire lock, nor by eyelash samples. In fact, in order to be able to operate correctly, the programs which are allocated to the analysis must operate on images relating to fibers which are distinct from one another. This aspect makes the process of preparation of the sample difficult and complicated, and is not easily transferable in the hands of inexpert operators.

Equipment has also been patented which can potentially combine both the approaches. An example of a strategy of this type is described in DE 10 2004 037566 A1. This document describes equipment which is provided with an optical measuring device, and can simultaneously carry out traction tests on a sample of hairs. Leaving out of consideration the objective difficulty both of miniaturizing this invention and making it easily and effectively usable in vivo, it is also important to emphasize that, in fact, it does not lend itself to the analysis of samples comprising a series of filaments simultaneously, since it is designed mainly to carry out traction tests on samples of single skin appendages (for example single hairs).

In order to be able to use this equipment in these contexts, accessories and protocols have been developed and adapted which are suitable for the most varied requirements. However, although this equipment is extremely precise and accurate, it is characterized by some extremely significant limits. These limits include above all:
- the high cost both of the machine itself and the accessories;
- the impossibility of use in vivo;
- the need for highly qualified personnel, which is indispensable for correct use and operation;
- costly processes of calibration and adaptation;
- heavy weight and large size.

In this context, it is particularly important to be able to have user-friendly equipment which is easy to transport, has a limited cost, and can combine simplicity of use with the possibility of obtaining realistic and reproducible qualitative-quantitative data both in vitro and in vivo, also using non-specialized personnel, and in the most varied operative contexts, ranging from a beauty salon to an experimental clinical laboratory.

The objective of the present invention is to make available instrumentation suitable for fulfilling the need for quantitative-quantitative evaluation of the surface characteristics, of some mechanical properties, and, indirectly, of some structural properties of filaments, in particular of vital or reconstituted tissues, in particular of hairs and eyelashes, whether they are vital or reconstituted, or of structures associated therewith, such as, by way of non-limiting example, artificial eyelashes, or evaluation of the effect of formulations for reconstruction or extension, and/or with treatment and/or decorative functions.

A further objective of the invention is to make available instrumentation which can permit the execution of measurements for the qualitative-quantitative characterization of filaments, and more particularly hairs and eyelashes, also, but not limited to, "in vivo" with non-invasive operations, for the implementation of protocols of characterization with a variable duration and in diversified conditions.

According to the present invention, these objectives are achieved thanks to a system for the characterization of vital or reconstituted tissues, in particular skin appendages such as hairs or eyelashes or structures associated therewith, having the characteristics indicated in claim 1.

Particular embodiments form the subject of the dependent claims, the content of which is to be understood as an integral part of the present description.

To summarize, the present invention is based on the principle of creating an aggregated characterization system, suitable for the study of filaments, in particular, but not limited to, hairs or eyelashes, using conservative methods associated with the acquisition of digital images which make it possible to monitor the analysis processes continually.

The operation of the characterization system which is the subject of the invention is based on the computerized analysis of digital images relating to phenomena of reflection and/or dispersion and/or fluorescence obtained by irradiating with a predetermined source of electromagnetic radiation, for example a source of light radiation, a prepared sample, positioned and optionally treated for this purpose. A prepared sample is a series of homogenous filaments per structural type, such as, by way of non-limiting example, a lock of hairs or a series of eyelashes of a person.

According to a particular embodiment, the correct positioning of the sample relative to the characterization system is obtained by using a gaseous flow, for example, but not limited to, air.

According to a further embodiment, the correct positioning of the sample relative to the characterization system is obtained thanks to dragging surfaces associated with the system. In particular contexts, the invention in question can also constitute an accessory of equipment which is already in use, such as, but not limited to, tensiometers.

This system can acquire data and digital images also of materials which cannot be removed during the stages of evaluation and study, and can therefore also operate in vivo and in situ. This advantageously permits the development of qualitative-quantitative evaluation protocols.

The process of analysis of the data and digital images acquired is carried out thanks to the use of a processor program executed by a control unit which is integrated in the system or is associated therewith, including remotely, based on the analysis of digital images acquired by means of an acquisition device created according to the teaching of the present invention. According to the teaching of the present invention, in accordance with a particular embodiment, there may also be provided a device for tensioning of the sample being examined, comprising a roller or a similar support rotating at a defined and controllable speed which, during the movement of rotation in contact with a sample to be examined, for example in the movement of sliding on the surface of the hairs of a sample lock, encounters a friction determined by the structure and surface condition of said sample.

In these particular operative contexts and given applications, the system can be provided with supplementary structures, which will be described hereinafter, being able to evaluate, whether in vivo or not, also the forces of friction, sliding or rolling of suitably designed probes relative to filaments, to skin appendages (in particular, but not limited to, hairs and eyelashes) and other structures, which structures are also developed for the purpose of simulating biological structures, such as, for example, but not limited to, locks of hair, artificial or reconstituted eyelashes, etc.

This therefore makes available a system for analysis in continuum which does not interfere with the processes to be evaluated in progress, which system can also operate in vivo and without damaging the sample in question.

The extreme simplicity of use and ease and immediacy of reading of the data produced make the instrument also particularly suitable for, but not limited to, demonstrative use, for example in a beauty salon, not requiring for its operation particularly specialized personnel or particular environmental conditions.

Further characteristics and advantages of the invention will be described in greater detail in the following detailed description of an embodiment thereof, provided by way of non-limiting example, with reference to the appended drawings, in which:
fig. 1 shows schematically a characterization system according to the invention;
figs. 2a and 2b show schematically a characterization system according to the invention according to a first embodiment;
fig. 3 shows three acquired images relating to a sample S of hairs illuminated respectively with natural light (a), red laser light (b), green laser light (c);
figs. 4a and 4b show schematically a characterization system according to the invention in a second embodiment;
figs. 5a and 5b show an exemplary embodiment of an element for support of the sample;
figs. 6a and 6b show an exemplary embodiment of an arrangement of an element for support of the sample, means for blocking/anchoring the sample, and of an element for tensioning or alignment of the sample;
figs. 7a and 7b show an exemplary embodiment of means for blocking/anchoring the sample;
fig. 8 shows an exemplary embodiment of an element for tensioning or alignment of the sample;
figs. 9a-9f show the results of the processing of images relating to different types of samples subjected to different illuminations;
fig. 10 shows images of reflection or dispersion from a sample irradiated with a source of green light; and
fig. 11 shows two images of a model of reconstituted eyelashes, irradiated respectively by a green (A) and red (B) source.

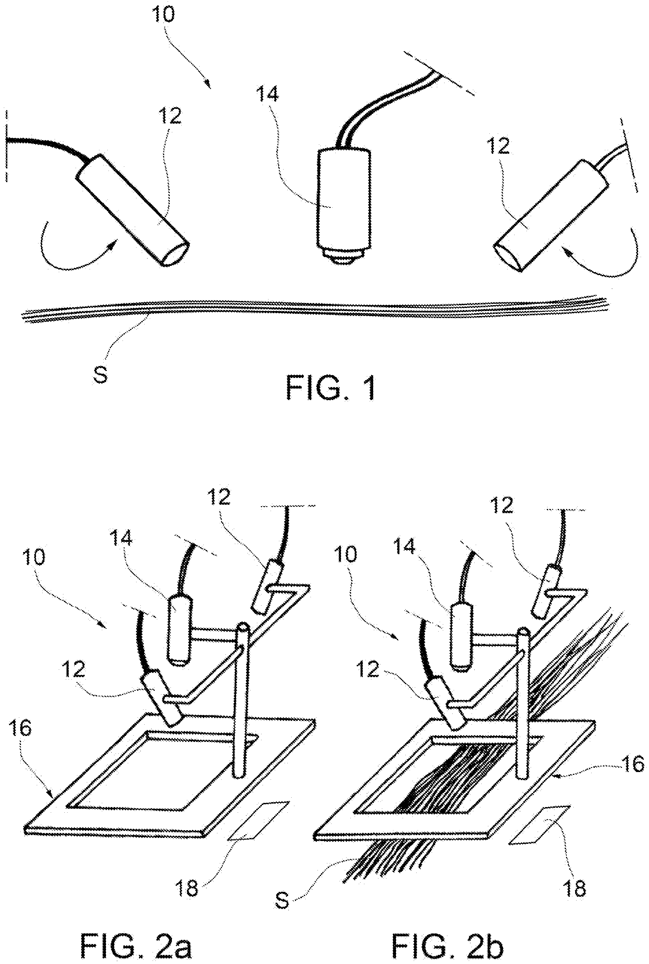

Figs. 1 and 2 show a characterization system, indicated as 10 as a whole, according to a first embodiment of the invention, in which the essential components are present.

The system comprises:
- at least one source of electromagnetic radiation 12, two thereof being shown in the figures;
- at least one image acquisition device 14; and
- means 18 for processing of the images.

The source of electromagnetic radiation (light and/or not light) 12 permits the generation of figures of reflection/dispersion/fluorescence by a sample S illuminated by an analysis radiation. This can include a source of incoherent light (for example, but not limited to, a white light LED source or any visible or non-visible wavelength) or a source of polarized light. The source of electromagnetic radiation includes a source of coherent light (for example a laser), characterized by a given wavelength. These sources can optionally also have a frequency suitable for the generation of phenomena of fluorescence on the irradiated sample.

Different images of reflection/dispersion/fluorescence of the sample S can correspond to different sources of radiation.

Optionally, the intensity of the sources of radiation can be regulated, and in this case different images of reflection/dispersion/fluorescence of the sample S can correspond to different electromagnetic flows.

Different sources can also be present simultaneously, and can preferably be oriented as required relative to the sample S. Alternatively, they are present selectively, and it is possible to replace or exchange them as required for the purpose of being able to carry out the selection of the most appropriate source of radiation in the shortest possible time.

According to a preferred embodiment, the sources can advantageously be directed, focused, and their intensity can be regulated independently, so as to adopt a predetermined angle of incidence of the radiation emitted on the sample. Different images of reflection/dispersion/fluorescence can correspond to different angles of incidence.

At least one source of coherent light (laser) is used, with planar geometry which can irradiate the sample along a linear section thereof, perpendicularly to the longitudinal axis of the fibers which constitute it. A source of this type with planar geometry irradiates the sample with a "blade" of light. When the "blade" of light meets the sample, it is partly reflected, partly passes through the sample, and is partly dispersed both on the surface and in the interior thereof. The whole of these phenomena gives rise to an image of the sample.

The image acquisition device 14 is designed to capture images generated by the irradiation of the sample by the source(s), using for example, but not exclusively, a digital camera or a digital microscope depending on the necessary level of enlargement required by the analysis. The level of enlargement can be varied as required according to the type of analysis in progress, and different digital acquisition apparatuses, including of different types, can be present simultaneously. Advantageously, according to a preferred embodiment, it is also possible to vary the point of observation of the sample by varying the position of the acquisition device 14. It is possible to capture both individual images of the sample, and films. Advantageously, the images which are captured are saved and processed directly by the system by the processing means 18, or alternatively they can be sent to external processing means.

A structure 16 for delimiting the area of acquisition of the images can be present, and, according to a less complex embodiment, comprises a support base characterized by the presence of an opening, optionally with a variable size, corresponding to the field of view of the image acquisition device. This structure can also advantageously contribute to maintaining the position of the sample during the acquisition of the images.

The processing means 18 are configured by means of a processor program which can execute a qualitative-quantitative analysis of the images and/or of the films captured. This analysis can be carried out directly, immediately after the acquisition of the images, or at any subsequent moment.

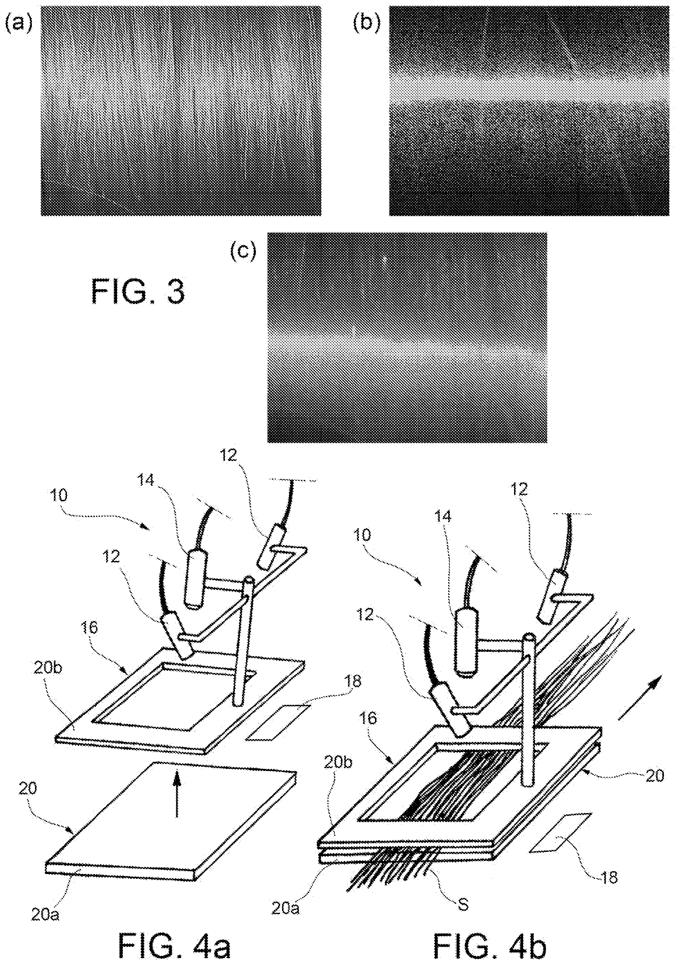

Figure 3 shows as an example the acquisition of the images relating to a sample S of hairs illuminated respectively with natural light (a), red laser light (b), green laser light (c).

Specifically, the processor program which exists in the processing means 18 is designed to analyze digital images of filaments, for example, but not limited to, skin appendages, in particular hairs and eyelashes, irradiated with different types of optical radiation, such as, for example, but not limited to, laser radiation, and acquired by means of, for example, but not limited to, a microscope or another acquisition device.

The analysis is carried out by means of specific algorithms suitable for the qualitative-quantitative definition of parameters such as gloss, size, colour, and indirectly the mechanical properties, in order to acquire information on the state of the sample, for example a lock of hair, and on the effect produced by any treatment carried out on the hair.

The analysis is carried out using known algorithms systems, such as Kayyali, Kirsch and Laplacian (3 x 3, 5 x 5 and Gaussian), examining a non-linear area created from amongst all the minimum and maximum image comparison peaks. A stage of presetting of the analysis procedure is dedicated to the acquisition of all the images, and the data acquired is used for construction of homogenous classes wherein each new sample can be classified; each of these classes can correspond to a series of parameters for the definition of analysis protocols. Thus, all the images acquired by means of the different forms of lighting, for a single type of sample, for example a lock of hair, form a preselection.

Advantageously, the processor program has a record section for storage of the record data of the people tested, so that in this section it is possible to consult and control all the analyses carried out and start a new analysis by acquiring the images necessary and selecting the preselection for the purpose of comparing the analyses.

By taking into consideration a historic development of the analysis results, it is possible to understand whether a treatment of the sample has produced an effect.

The archive of the tests carried out can be saved in order to have a historic development of the treatments and of their effects on the sample, for example hair, and to constitute a database.

The database can be supplied via the Internet to all the users so as to improve the statistical reliability of the results obtained.

The analysis of the sample, for example hairs, is carried out in an image analysis stage, by loading the images and comparing them with the corresponding reference classes previously defined, or with the images relating to the same person or sample, acquired at different times. During this stage of the program, it is possible to carry out the analysis without necessarily having to identify the person to whom the sample belongs.

Each analysis is carried out on the basis of the predefined parameters. For example, an analysis can be carried out on the basis of the percentage of similarity of the sample to the reference image, preferably using the following parameters:
scale = brightness
colour = intensity and gloss of colour
contour = details of the filaments.

In its definitive version, the processor program can be integrated directly inside a portable device, and show only the result on a display unit which is connected directly. Alternatively, the device can send the data captured wirelessly to a PC which will carry out the analysis and upkeep of the data itself.

The analysis of the sample is not necessarily based on the separation, characterization and quantification of the various fractions of radiation after the interaction with the sample. Advantageously, it is not necessary, for example, to establish and quantify the fraction of radiation which is reflected by the surface of the sample, or the fraction which passes through it, but it is the image of the sample as a whole which is informative.

The real and extreme structural simplification of most of the embodiments of the present invention is associated with a real possibility of miniaturization, a fundamental aspect which permits simple and widespread use thereof, in particular for applications in vivo, as well as a restriction of the production costs.

In different embodiments, the system has additional elements which provide further characteristic functions.

According to a second embodiment, the system according to the invention also includes a region 20 for alignment and stretching of the sample, and a grasping system (not illustrated) which is equipped with devices for command and control of the system, as shown in figs. 4a and 4b.

The region 20 for alignment and stretching comprises for example a support 20a and a gripping frame 20b. The sample S is extended or stretched on the support 20a which permits homogenous, flat and continuous distribution thereof. In order to assist this process, if the sample is constituted by fibers of a significant length, such as, for example, but not limited to, fibers or hairs, it can be advantageous to make the region 20 for alignment and stretching, or a part thereof, slide along the sample, thus assisting the distribution and stretching thereof, including optionally integrally with the region 20b.

The selection of the sample is greatly simplified since it is not necessary to select one fiber at a time, or a few fibers which are well separated and aligned with one another. On the contrary, in the most demanding hypothesis, it is sufficient to select a sample which is quantitatively sufficient to cover a defined surface, in the most stringent cases operating on the basis of a criterion of "minimum quantity" necessary to guarantee the cover of a given surface (for example in the case of analysis of locks of hair both in vivo and ex vivo) whereas in fact in other cases (such as, for example as far as the study of eyelash samples is concerned) it is sufficient to include in the field of irradiation an arbitrary number of elements of the sample in relation to which the analysis is being carried out.

The grasping system comprises the devices necessary for the operation of the system according to the invention, including control of the region of alignment and stretching, as well as the commands relating to, for example, but not limited to, switching on/switching off of the sources of light, capture of the images/films, etc. Optionally, there can also be a display unit for direct display of the region of alignment and of the images/films acquired.

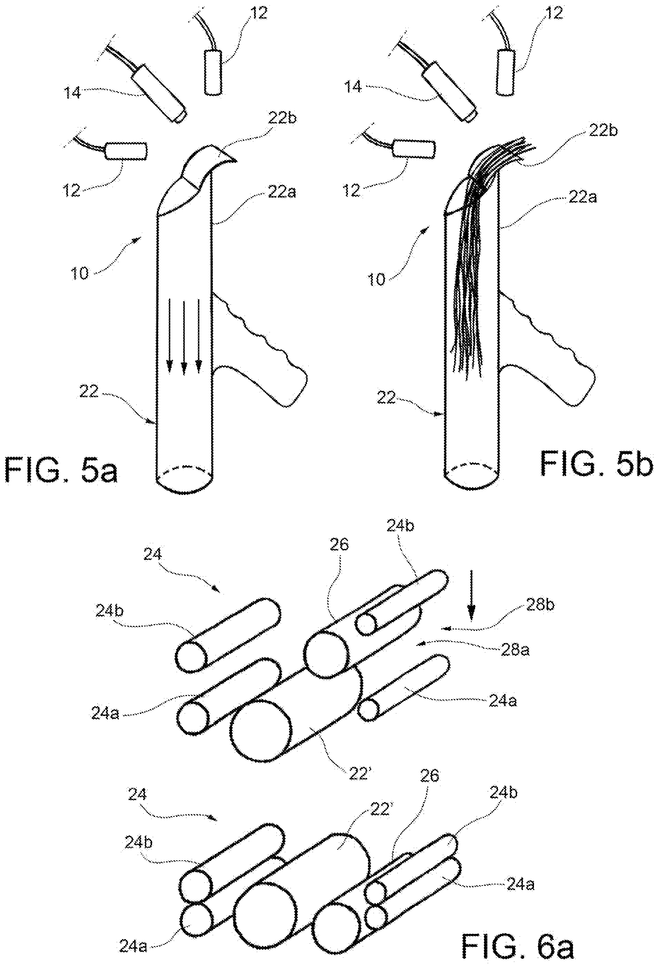

According to a third embodiment, as shown in figs. 5a and 5b, the system which is the subject of the invention also includes:
- an element 22 for support of the sample; and
- means for blocking/anchoring the sample (preferably for "ex vivo" evaluations).

The sample in question can optionally be distributed prevalently in a generally planar region, so as to avoid where possible the presence of different focal planes. This objective can be achieved in various ways, some of which are cited hereinafter.

The sample in question can advantageously be supported and/or distributed on a homogenous surface which can include a flat or curved surface.

The colour as well as the roughness and/or gloss of the surface of the support element is of significant importance. A non-limiting example of a structure of this type is represented in fig. 5a. According to a simplified embodiment, the distribution and alignment of the sample are implemented directly by a thrust or suction flow generated or conveyed in a stretching region of the element 22, and an at least partly exterior curved surface constitutes the analysis surface.

According to the present embodiment, a process of stretching and tensioning of the sample could be advantageous. In this case, this process is carried out by a vacuum source (not illustrated) which subjects the sample to tension by suctioning it, or alternatively blowing it, in the interior of a conduit 22a with an appropriate geometry, thus giving rise to the adhesion and distribution on the analysis surface 22b of the support element 22, as shown in fig. 5b. More particularly, this support element 22 can be constituted by a structure 22a, which for example is cylindrical, and is preferably, but not necessarily, transparent, connected for example to a suction device, to a Venturi tube, etc., and by a curved surface 22b which provides the analysis surface.

The sample can be blocked by mechanical means, in particular, but not necessarily, during the "in vitro" analysis, which means secure it in an appropriate position and prevent undesirable movements thereof. These means can for example, but not necessarily, include a pair of jaws which block the sample, closing it in the form of a clamp. By way of non-limiting example, these jaws can comprise two parallelepipeds or two simple opposing rollers or cylinders. Optionally, these jaws can have a geometry which can also assist and guarantee homogenous distribution of the sample during the clamping and blocking stage.

According to a further embodiment, the system includes:
- an element for support of the sample comprising at least one roller or cylinder 22';
- means for blocking/anchoring the sample comprising a pair of lower rollers or cylinders 24a and a pair of upper rollers or cylinders 24b; and
- an element 26 for tensioning/alignment of the sample, comprising a roller.

The roller or cylinder 22' for support of the sample, together with the pair of lower rollers or cylinders 24a of the means for blocking/anchoring the sample, forms a first arrangement of elements 28a, whereas the roller 26 for tensioning or alignment of the sample, together with the pair of lower rollers or cylinders 24b, forms a second arrangement of elements 28b, which is at least partly interpenetrable with the first device 28a.

Fig. 6a shows the first and second arrangement of elements 28a, 28b, respectively in an "open" configuration and in a "closed" configuration, i.e. interpenetrated, which arrangement can be obtained further to a relative movement of the arrangements according to the arrow in the figure. Each arrangement of elements is preferably a rigid arrangement consisting of three elements, i.e. an arrangement wherein the three elements maintain a mutual spatial position which is unvaried in the translation from the "open" configuration to the "closed" configuration.

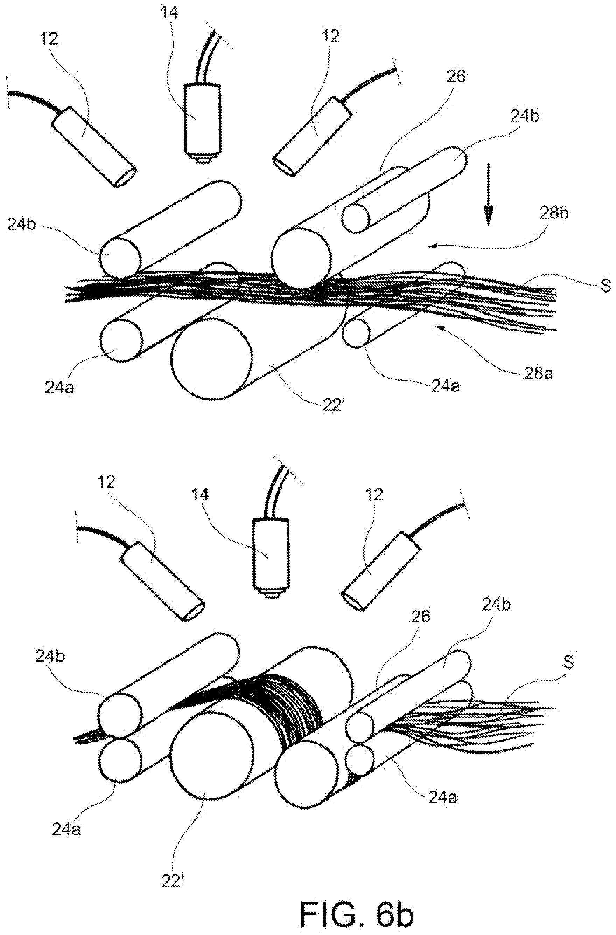

In fig. 6b, the first and second arrangements of elements 28a, 28b are shown in respective "open" and "closed" conditions relative to a sample S.

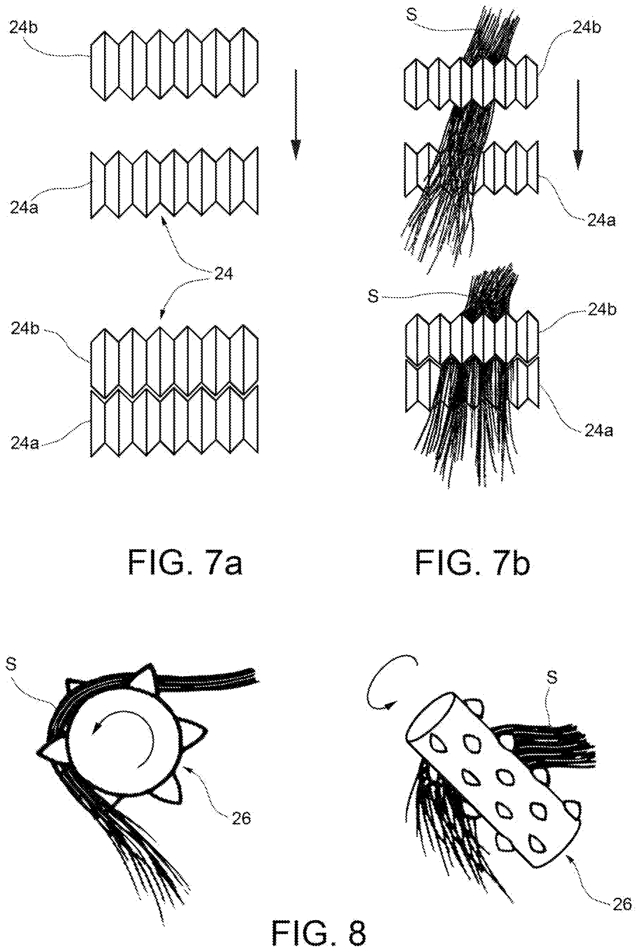

An exemplary, but non-limiting embodiment of the means 24 for blocking/anchoring the sample is illustrated in figs. 7a and 7b. In this example, in the lateral surfaces of the rollers or cylinders 24a, 24b, there are provided grooves, which for example have a triangular cross-section, which, during the closure and blocking of the sample, engage in a complementary manner, thus both guaranteeing adequate securing, and contributing towards distribution of the sample along the clamping surface, thus acting as a type of comb.

According to a simpler embodiment, these jaws can have a parallelepiped cross-section, including with beveled edges.

In all cases, these elements or the surfaces in direct contact with the sample are preferably constructed of, or covered with, appropriate friction material which can guarantee an excellent hold, without causing any damage to the sample itself.

Optionally, but not necessarily, in order to be able to be subjected to analysis, the sample must be characterized by a certain parallelism of the elements which constitute it along their major axis. Sometimes this situation is already intrinsic in the sample (for example if a portion of eyelashes is taken into consideration, these can be approximately parallel to one another).

This characteristic need not be intrinsic in the sample itself (if account is taken for example of a lock of hairs which are curly or tousled). For this reason, the support element can optionally, but not necessarily, be coupled to an element which assists the tensioning and alignment of the elements which constitute the sample. Typically, but not necessarily, this element can comprise a rotary structure, such as, for example, the cylinder 26, shown in detail according to a lateral view and a view in perspective in fig. 8, or a prism which for example, but not necessarily, has a hexagonal base.

In order to generate a surface of the sample with characteristics suitable for the analysis, the surface of the tensioning/alignment element is characterized by a particular geometry (also determined by the cross-section of the prism), and can be constituted by, or coated with, a particular friction material which, by engaging on the sample, determines the stretching and optimum distribution thereof on the analysis surface. Typically, but not necessarily, the action of tensioning or alignment is carried out by means of the rotation of this element about its central axis (fig. 8). This rotation can be implemented for example by means of a gearmotor, the actuation of which is at the discretion of the operator carrying out the analysis. Optionally, it is possible to measure the force exerted by the tensioning or alignment element for rotation, thus obtaining information concerning the friction of the sample being analyzed relative to this element.

By way of example, but not necessarily, this measurement can be made by means of measurement of the power absorbed by the gearmotor which actuates the tensioning/alignment element in order to start and keep it rotating.

If it is present, the tensioning/alignment element 26 is typically, but not necessarily, located on the side opposite the blocking/anchoring means 24, with the support element 22 interposed between the two. Its action contributes towards generating an appropriate analysis surface.

Alternative embodiments can be considered, which have the characteristics forming the subject of the present invention, for example for analysis of artificial samples (such as, for example, but not limited to, false eyelashes or synthetic fibers) or of samples obtained ex-vivo (such as, for example, but not limited to, eyelashes reconstituted using natural skin appendages such as animal hairs or other natural fibers, or locks of hair, etc.).

For use of this type, the system which is the subject of the invention need not necessarily be manageable manually, and optionally can thus be produced so as to be able to constitute a workbench apparatus which, although it is easy to transport, is used in a stationary manner.

In order to reduce the production costs and simplify the characteristics of use thereof, this embodiment can optionally be simplified. In particular, optionally, it would be possible to eliminate the tensioning or alignment element from the system. This simplified solution would be advantageous in the case of, for example, but not limited to, the study of samples of fibers (such as, for example, artificial or reconstituted eyelashes), the short length of which would make the tensioning/alignment element unnecessary.

### Example 1: Constitution of reference classes by means of image analysis

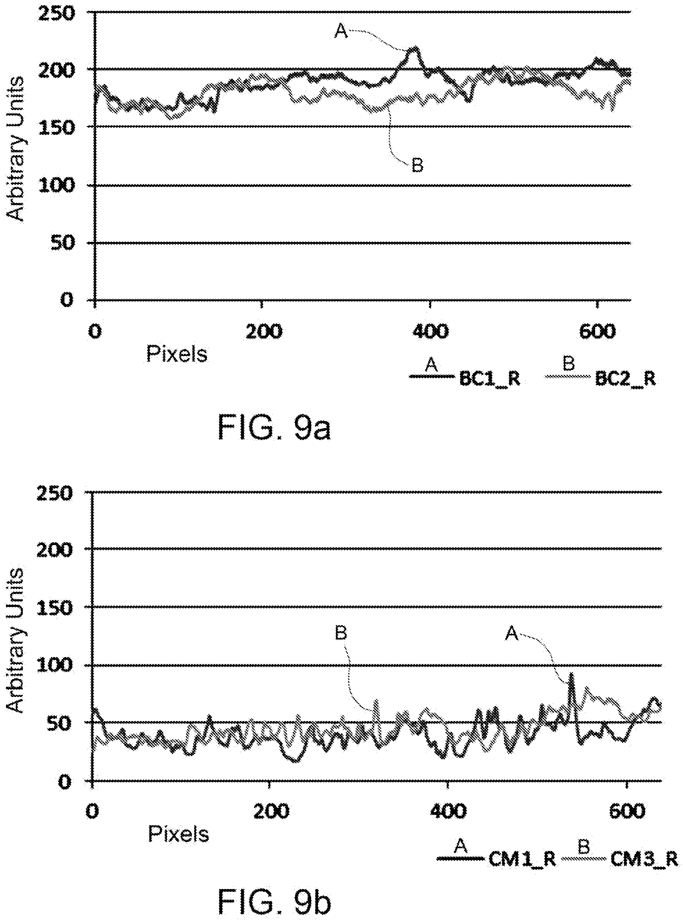

In an example of application, the system according to the invention was used to determine reference classes by means of image analysis, for example, but not necessarily, in order to classify samples of hairs on the basis of colour. For this purpose, 4 groups were prepared constituted by 8 locks each, respectively of a light blonde colour (BC), strawberry blonde (BR), light brown (CC) and medium brown (CM). By way of example, figs. 9a-9f contain the results of processing of some images relating to different types of locks, analyzed with different types of lighting, respectively:
- in fig. 9a, lighting with red laser of two BC locks (curve A and curve B);
- in fig. 9b, lighting with red laser of two CM locks (curve C and curve D);

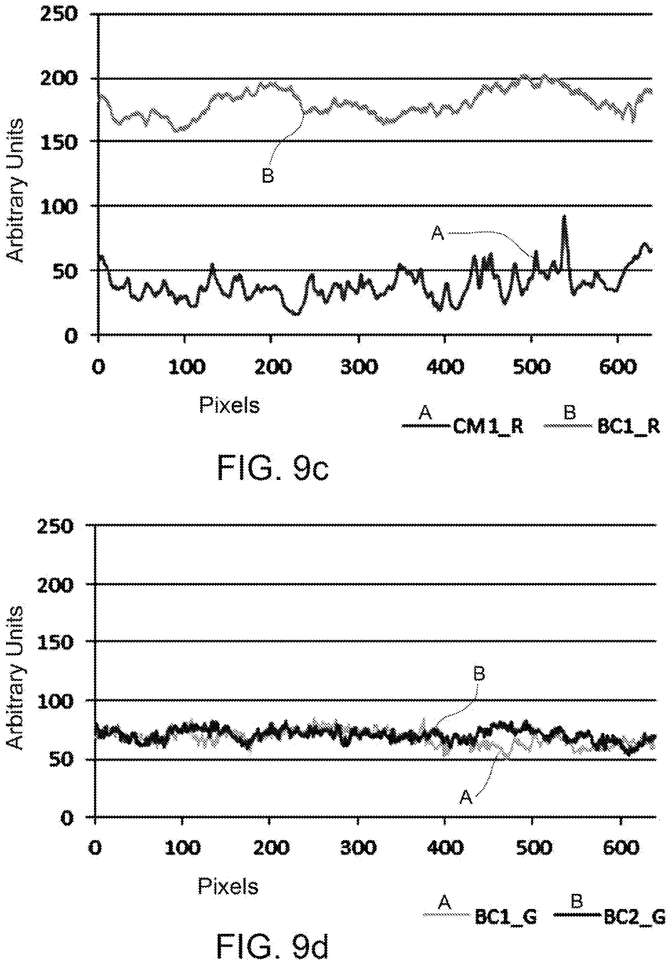

- in fig. 9c, lighting with red laser and comparison between a CM lock and a BC lock (curve C and curve B);
- in fig. 9d, lighting with green laser of BC locks (curve E and curve F);

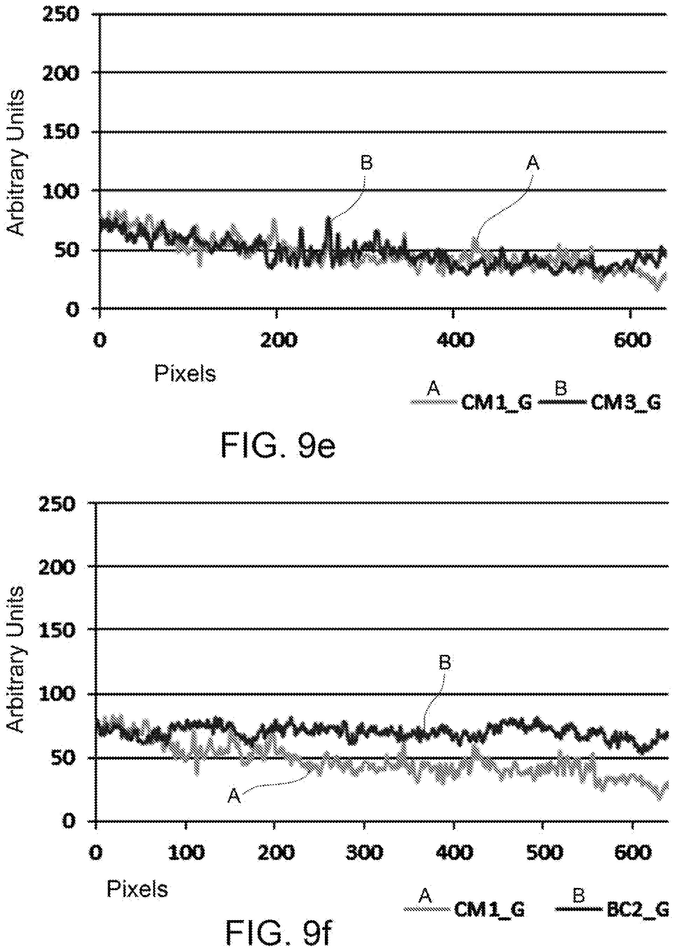

- in fig. 9e, lighting with green laser of CM locks (curve G and curve H);
- in fig. 9f, lighting with green laser and comparison between a CM lock and a BC lock (curve G and curve F).

### Example 2: Effect of a treatment which worsens the characteristics of the hair

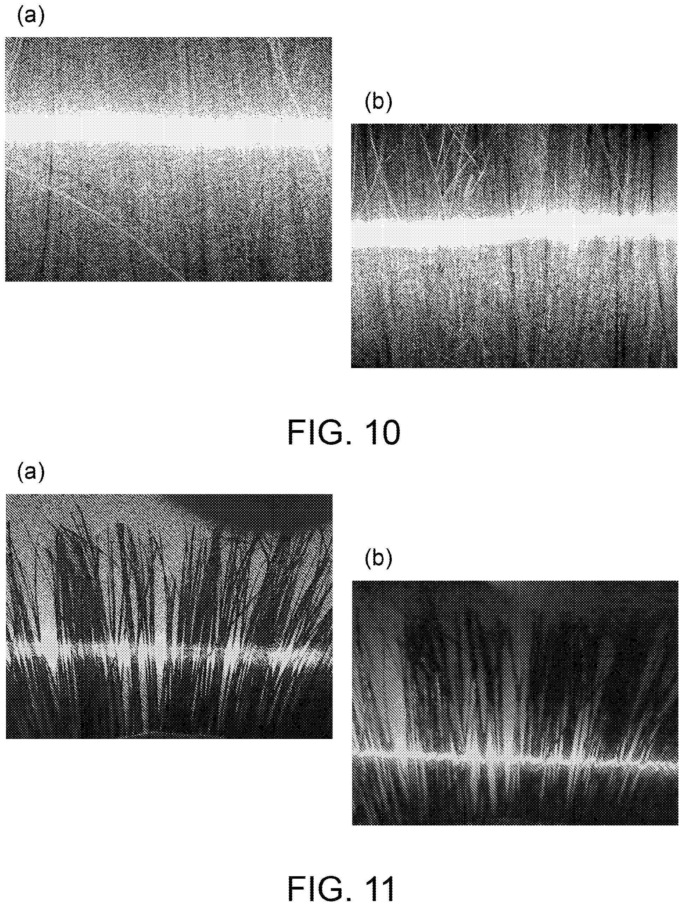

In order to give an example of the effectiveness of application of the system which is the subject of the invention in determining a modification of the optical response of a sample, for example for analysis of the effects of a treatment which worsens the characteristics of a hair, locks of hair as such (natural), and locks after reducing chemical treatment were analyzed. The results showed a radical change of the type of reflection/dispersion, as shown in fig. 10, in which the sample is irradiated with a source of green light. The obvious qualitative differences which can already be ascertained visually were confirmed and quantified by means of the image analysis.

Analysis of the sample treated chemically and irradiated with various sources (green and red) gave results relating to the parameters, for example but not limited to, Scale, Chromatic and Contour, which can be summarized as a percentage of similarity (score) with respect to the reference (natural hair). Below, by way of example, the results are given of the aforementioned analysis, from which it is deduced that chemical treatment gives rise to variation of the score of more than 25%.

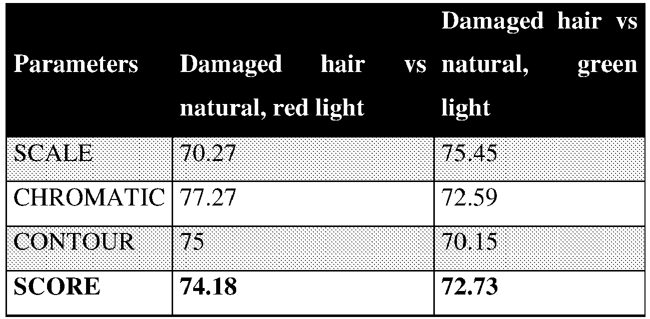

This variation is comparable with respect to the two sources used. These results confirm the capacity of the system according to the invention to show structural variations of the surface of the sample.

### Example 3: Effect of a conditioning cosmetic treatment

In order to give an example of the effectiveness of application of the system wh according to the invention in determining the effect of a treatment, for example, but not necessarily, a cosmetic treatment, analysis was carried out of locks of damaged hair and locks after cosmetic treatment with a conditioner. The table is given below, with the corresponding reprocessing of the samples irradiated with a red and green source.

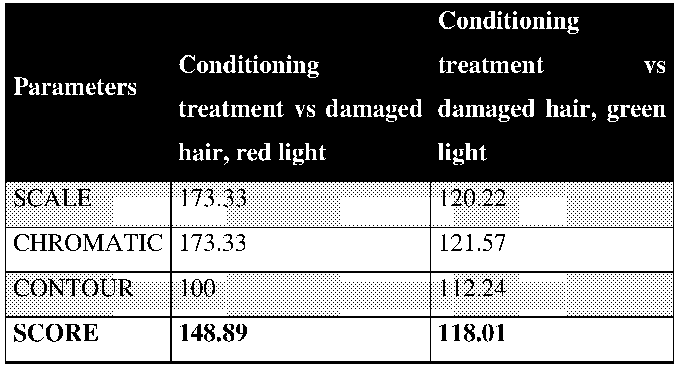

The results show an improvement of the morphological characteristics of the sample which results in increase of the score of more than 15%. The results obtained on various locks, including those irradiated with various sources, were reproducible, and therefore confirm the reliability of the data produced by use of the invention.

### Example 4: Evaluation of eyelashes

The analysis of short filaments, such as, for example, but not necessarily, eyelashes, can be carried out with the system according to the invention, both in vivo and on models constructed ad hoc in order to evaluate the surface characteristics thereof which can be attributed to the reflection/dispersion images, as well as some specific parameters such as, for example, the thickness, the definition, the curvature and the linear density.

Fig. 11 shows two images of a reconstituted eyelash model, irradiated respectively by a green (A) and red (B) source.

The image processing makes it possible to obtain a digital image profile, from which parameters of interest can be obtained, by applying appropriate algorithms.

### Example 5: Comparative evaluation of the performance levels of two applicators for mascara

The system according to the present invention was used to evaluate the performance levels of two different applicators for mascara in terms of homogeneity of distribution of the product. For this purpose, samples of a reconstituted eyelash model were prepared and divided into two groups (A and B). The same make-up product (mascara) was applied to each group, using two different applicators (A and B). The analyses were carried out in triplicate.

The first analysis carried out in order to evaluate the homogeneity of the eyelash samples prepared, the results of which are given in the following table, indicates that the samples do not differ from one another, and have a score of approximately 100%.

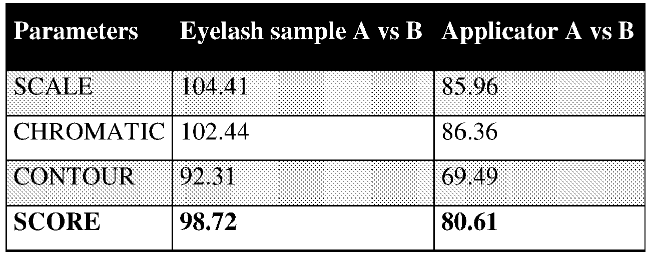

This result is fundamental for evaluating subsequently the effect of the use of the different applicators on samples which are different, but homogenous relative to one another.

The table contains for example the results obtained for the artificial eyelash samples, with evaluation of the Scale, the Chromatic and the Contour. The results show a substantial difference between the two samples, emphasizing the different performance levels of the two applicators. The similarity index (score) is reduced by almost 20%.

It will be appreciated that, whilst retaining the principle of the invention, the embodiments and details of implementation can be widely varied compared with those described and illustrated purely by way of non-limiting example, without however departing from the scope of the invention defined by the appended claims.

## Claims

1. A system for the characterization of vital or reconstituted tissues, in particular skin appendages such as hair or eyelashes or structures associated therewith, wherein it comprises in combination:
- a plurality of sources (12) of electromagnetic radiations of analysis with different wavelengths adapted to irradiate a sample (S) of vital or reconstituted tissue comprising a series of filaments of a person which are homogeneous in their structural type, including one of a lock of hairs or a series of eyelashes of a person, that are homogeneously, flat and continuously distributed on a support said plurality of sources including at least one source of coherent light with planar geometry which is arranged to irradiate the sample along a linear section thereof;
- at least one image acquisition device (14) arranged in a region close to the sample (S) so as to receive a reflected and/or dispersed radiation from the sample (S) when irradiated by said electromagnetic radiation of analysis and/or a fluorescence radiation emitted by the sample (S) due to the effect of said electromagnetic radiation of analysis; and
- image processing means (18) designed to process reflection and/or dispersion images of said electromagnetic radiation from the sample (S) and/or fluorescence images emitted by the sample (S) due to the effect of said electromagnetic radiation,
wherein the angle of incidence of the radiation on the sample form said sources (12) of electromagnetic radiation of analysis is adapted to be selected relative to the sample (S) and said at least one source of coherent light with planar geometry is arranged to irradiate the sample along a linear section perpendicular to the longitudinal axis of the filaments which constitute said sample; and
wherein said image processing means (18) are further designed for:
- determining at least one of the parameters out of gloss, size, colour, contour of the filaments of the sample (S) of said tissue starting from said acquired images and for classifying the tissue under examination by comparison of the value of said at least one parameter with a database of parameters of classes of predetermined vital or reconstituted tissues,
and/or
- determining at least one out of the parameters of gloss, size, colour, contour of the filaments of the sample (S) of said tissue starting from said acquired images and for comparing the value of said at least one parameter with a database of historic values of said at least one parameter for said tissue.

2. The system as claimed in claim 1, further comprising a structure (16) for delimiting an image acquisition area, comprising at least one support base which has an opening in correspondence with the field of view of said at least one image acquisition device (14).

3. The system as claimed in claim 2, further comprising sample positioning means (S) adapted to arrange said sample (S) within said image acquisition area, wherein said sample positioning means (S) comprise a region (20) for aligning the sample (S).

4. The system as claimed in claim 3, wherein said region (20) for aligning the sample (S) comprises a support (20a) adapted to receive the sample (S) in an extended condition and a gripping frame (20b), and is slidable at least partly along the sample (S).

5. The system as claimed in claim 3, wherein said region (20) for aligning the sample (S) comprises a conduit adapted to support a gaseous flow for thrusting or suctioning the sample (S).

6. The system as claimed in any one of the preceding claims, further comprising means (22) for supporting the sample (S) arranged facing said at least one image acquisition device (14).

7. The system as claimed in claim 6, wherein said sample support means (22) (S) have a homogeneous flat or curved surface.

8. The system as claimed in claim 7, wherein said sample support means (22) (S) comprise at least one roller or cylinder (22').

9. The system as claimed in any one of the preceding claims, further comprising means (24) for blocking/anchoring the sample (S) adapted to hold the sample (S) in a predetermined position with respect to said at least one source of electromagnetic radiation (12) and to said at least one image acquisition device (14).

10. The system as claimed in claim 9, wherein said blocking/anchoring means (24) of the sample (S) comprise at least a pair of opposing clamping elements (24a, 24b) adapted to clamp the sample (S) between them.

11. The system as claimed in claim 10, wherein said at least one pair of clamping elements (24a, 24b) comprises a pair of lower rollers or cylinders (24a) and a pair of upper rollers or cylinders (24b) adapted to come into contact with each other in a clamping condition of the sample (S).

12. The system as claimed in any one of claims 10 or 11, wherein the lateral surfaces of said clamping elements (24a, 24b) have alternate grooves and reliefs which in the clamping condition of the sample (S) engage in a complementary way, so as to assist a transversely homogeneous distribution of the sample (S) during clamping.

13. The system as claimed in any one of claims 10 to 12, wherein said at least one pair of clamping elements (24a, 24b) has contact surfaces with the sample (S) having a coating of a material adapted to make friction against the sliding of the sample (S).

14. The system as claimed in any one of the preceding claims, further comprising means (26) for tensioning the sample (S), wherein said sample tensioning means (26) preferably comprise at least one roller adapted to rotate about its own axis, the lateral surface of which is coated with a material adapted to make friction against the sliding of the sample (S).

## Patentansprüche

1. System zur Charakterisierung von vitalen oder wieder aufgebauten Geweben, insbesondere von Hautanhangsgebilden wie Haaren oder Wimpern oder damit verbundenen Strukturen, wobei es in Kombination enthält:
- eine Vielzahl von Quellen (12) elektromagnetischer Analysestrahlungen mit unterschiedlichen Wellenlängen, die angepasst sind zum Bestrahlen einer Probe (S) von vitalem oder wieder aufgebautem Gewebe, das eine Reihe von Fasern einer Person umfasst, die in ihrem Strukturtyp homogen sind, einschließlich einer Haarlocke oder einer Reihe von Wimpern einer Person, die homogen, flach und kontinuierlich auf einem Träger verteilt sind, wobei die Vielzahl von Quellen mindestens eine Quelle kohärenten Lichts mit planarer Geometrie umfasst, die angeordnet ist zum Bestrahlen der Probe entlang eines linearen Schnitts davon;
- mindestens eine Bilderfassungseinrichtung (14), die in einem Bereich nahe der Probe (S) angeordnet ist zum Empfangen einer reflektierten und/oder gestreuten Strahlung von der Probe (S), wenn sie von der elektromagnetischen Analysestrahlung bestrahlt wird, und/oder einer Fluoreszenzstrahlung, die von der Probe (S) aufgrund der Wirkung der elektromagnetischen Analysestrahlung emittiert wird; und
- Bildverarbeitungsmittel (18), die entworfen sind zum Verarbeiten von Reflexions- und/oder Dispersionsbildern der elektromagnetischen Strahlung von der Probe (S) und/oder von Fluoreszenzbildern, die von der Probe (S) aufgrund der Wirkung der elektromagnetischen Strahlung emittiert werden,
wobei der Einfallswinkel der Strahlung auf die Probe aus den Quellen (12) der elektromagnetischen Analysestrahlung angepasst ist, dass er relativ zu der Probe (S) gewählt wird, und die mindestens eine Quelle kohärenten Lichts mit planarer Geometrie angeordnet ist zum Bestrahlen der Probe entlang eines linearen Abschnitts senkrecht zu der Längsachse der Fasern, die die Probe bilden; und
wobei die Bildverarbeitungsmittel (18) ferner ausgelegt sind zum:
- Bestimmen von mindestens einem aus den Parametern Glanz, Größe, Farbe, Kontur der Fasern der Probe (S) des Gewebes ausgehend von den erfassten Bildern und zum Klassifizieren des untersuchten Gewebes durch Vergleich des Wertes des mindestens einen Parameters mit einer Datenbank von Parametern von Klassen vorbestimmter vitaler oder wieder aufgebauter Gewebe, und/oder
- Bestimmen von mindestens einem aus den Parametern Glanz, Größe, Farbe, Kontur der Fasern der Probe (S) des Gewebes ausgehend von den erfassten Bildern und zum Vergleichen des Wertes des mindestens eines Parameters mit einer Datenbank historischer Werte des mindestens einen Parameters für das Gewebe.

2. System nach Anspruch 1, ferner enthaltend eine Struktur (16) zum Begrenzen eines Bilderfassungsgebiets, die mindestens eine Trägerbasis aufweist, die eine Öffnung in Übereinstimmung mit dem Sichtfeld der mindestens einen Bilderfassungseinrichtung (14) hat.

3. System nach Anspruch 2, ferner enthaltend Probenpositionierungsmittel (S), die angepasst sind zum Anordnen der Probe (S) innerhalb des Bilderfassungsgebiets, wobei die Probenpositionierungsmittel (S) einen Bereich (20) zum Ausrichten der Probe (S) aufweisen.

4. System nach Anspruch 3, wobei der Bereich (20) zum Ausrichten der Probe (S) einen Träger (20a), der zum Aufnehmen der Probe (S) in einem ausgefahrenen Zustand angepasst ist, und einen Greifrahmen (20b) aufweist und zumindest teilweise entlang der Probe (S) verschiebbar ist.

5. System nach Anspruch 3, wobei der Bereich (20) zum Ausrichten der Probe (S) eine Leitung aufweist, die angepasst ist zum Fördern eines Gasstroms zum Schieben oder Saugen der Probe (S).

6. System nach einem der vorhergehenden Ansprüche, ferner enthaltend Mittel (22) zum Tragen der Probe (S), die auf die mindestens eine Bilderfassungseinrichtung (14) ausgerichtet angeordnet sind.

7. System nach Anspruch 6, wobei die Probentragmittel (22) (S) eine homogene flache oder gekrümmte Oberfläche aufweisen.

8. System nach Anspruch 7, wobei die Probentragmittel (22) (S) mindestens eine Rolle oder einen Zylinder (22') aufweisen.

9. System nach einem der vorhergehenden Ansprüche, ferner enthaltend Mittel (24) zum Blockieren/Verankern der Probe (S), die angepasst sind zum Halten der Probe (S) in einer vorbestimmten Position mit Bezug auf die mindestens eine Quelle elektromagnetischer Strahlung (12) und die mindestens eine Bilderfassungseinrichtung (14).

10. System nach Anspruch 9, wobei die Blockier-/Verankerungsmittel (24) der Probe (S) mindestens ein Paar gegenüberliegender Klemmelemente (24a, 24b) aufweisen, die angepasst sind zum Klemmen der Probe (S) dazwischen.

11. System nach Anspruch 10, wobei das mindestens ein Paar von Klemmelementen (24a, 24b) ein Paar von unteren Rollen oder Zylindern (24a) und ein Paar von oberen Rollen oder Zylindern (24b) aufweist, die angepasst sind, dass sie in einem Klemmzustand der Probe (S) miteinander in Berührung kommen.

12. System nach einem der Ansprüche 10 oder 11, wobei die Seitenflächen der Klemmelemente (24a, 24b) abwechselnde Rillen und Erhebungen aufweisen, die im Klemmzustand der Probe (S) auf komplementäre Weise ineinandergreifen, sodass sie bei einer transversal homogenen Verteilung der Probe (S) während eines Einklemmens helfen.

13. System nach einem der Ansprüche 10 bis 12, wobei das mindestens ein Paar von Klemmelementen (24a, 24b) Kontaktflächen mit der Probe (S) aufweist, die eine Beschichtung aus einem Material aufweisen, die angepasst ist zum Erzeugen von Reibung gegen das Gleiten der Probe (S).

14. System nach einem der vorhergehenden Ansprüche, ferner enthaltend Mittel (26) zum Spannen der Probe (S), wobei die Probenspannmittel (26) vorzugsweise mindestens eine um ihre eigene Achse drehbare Rolle aufweisen, deren Seitenfläche mit einem Material beschichtet ist, das angepasst ist zum Erzeugen von Reibung gegen das Gleiten der Probe (S).

## Revendications

1. Système de caractérisation de tissus vivants ou reconstitués, en particulier des phanères tels que des cheveux ou des cils ou des structures associées à ceux-ci, dans lequel il comprend en combinaison :
- une pluralité de sources (12) de rayonnement électromagnétique d'analyse avec différentes longueurs d'onde adaptées pour exposer un échantillon (S) de tissu vivant ou reconstitué comprenant une série de filaments d'une personne qui sont homogènes dans leur type structural, notamment un échantillon parmi une mèche de cheveux ou une série de cils d'une personne, qui sont uniformément répartis, à plat et en continu, sur un support, ladite pluralité de sources incluant au moins une source de lumière cohérente avec géométrie plane qui est agencée de façon à exposer l'échantillon le long d'une section linéaire de celui-ci ;
- au moins un dispositif d'acquisition d'images (14) agencé dans une région proche de l'échantillon (S) de manière à recevoir un rayonnement réfléchi et/ou dispersé en provenance de l'échantillon (S) lorsqu'il est exposé audit rayonnement électromagnétique d'analyse et/ou à un rayonnement de fluorescence émis par l'échantillon (S) sous l'effet dudit rayonnement électromagnétique d'analyse ; et
- des moyens de traitement d'images (18) conçus pour traiter des images de réflexion et/ou de dispersion dudit rayonnement électromagnétique en provenance de l'échantillon (S) et/ou des images de fluorescence émises par l'échantillon (S) sous l'effet dudit rayonnement électromagnétique,
où l'angle d'incidence du rayonnement sur l'échantillon provenant desdites sources (12) de rayonnement électromagnétique d'analyse est adapté pour être sélectionné par rapport à l'échantillon (S) et ladite source de lumière cohérente avec géométrie plane, au moins au nombre de une, est agencée pour exposer l'échantillon le long d'une section linéaire perpendiculaire à l'axe longitudinal des filaments qui constituent ledit échantillon ; et
dans lequel lesdits moyens de traitement d'images (18) sont en outre conçus pour :
- déterminer au moins un des paramètres parmi la brillance, la dimension, la couleur, le contour des filaments de l'échantillon (S) dudit tissu en partant desdites images acquises et pour classifier le tissu examiné par comparaison de la valeur dudit paramètre, au moins au nombre de un, avec une base de données de paramètres de classes de tissus vivants ou reconstitués prédéterminés,
et/ou
- déterminer au moins un paramètre parmi les paramètres de brillance, dimension, couleur, contour des filaments de l'échantillon (S) dudit tissu en partant desdites images acquises et pour comparer la valeur dudit paramètre, au moins au nombre de un, avec une base de données de valeurs historiques dudit paramètre, au moins au nombre de un, pour ledit tissu.

2. Système tel que revendiqué dans la revendication 1, comprenant en outre une structure (16) permettant de délimiter une zone d'acquisition d'images, comprenant au moins une base de support qui présente une ouverture en correspondance avec le champ de vision dudit dispositif d'acquisition d'images (14), au moins au nombre de un.

3. Système tel que revendiqué dans la revendication 2, comprenant en outre des moyens de positionnement d'échantillon (S) adaptés pour positionner ledit échantillon (S) à l'intérieur de ladite zone d'acquisition d'images, dans lequel lesdits moyens de positionnement d'échantillon (S) comprennent une région (20) permettant d'aligner l'échantillon (S).

4. Système tel que revendiqué dans la revendication 3, dans lequel ladite région (20) permettant d'aligner l'échantillon (S) comprend un support (20a) adapté pour recevoir l'échantillon (S) dans un état étendu et un cadre de préhension (20b), et peut coulisser au moins partiellement le long de l'échantillon (S).

5. Système tel que revendiqué dans la revendication 3, dans lequel ladite région (20) permettant d'aligner l'échantillon (S) comprend un conduit adapté pour permettre la circulation d'un flux gazeux pour pousser ou aspirer l'échantillon (S).

6. Système tel que revendiqué dans l'une quelconque des revendications précédentes, comprenant en outre des moyens (22) permettant de supporter l'échantillon (S) positionné en face dudit dispositif d'acquisition d'images (14), au moins au nombre de un.

7. Système tel que revendiqué dans la revendication 6, dans lequel lesdits moyens de support d'échantillon (22) (S) présentent une surface homogène plate ou incurvée.

8. Système selon la revendication 7, dans lequel lesdits moyens de support d'échantillon (22) (S) comprennent au moins un rouleau ou un cylindre (22').

9. Système tel que revendiqué dans l'une quelconque des revendications précédentes, comprenant en outre des moyens (24) permettant de bloquer/immobiliser l'échantillon (S) adaptés pour maintenir l'échantillon (S) dans une position prédéterminée par rapport à ladite source de rayonnement électromagnétique (12), au moins au nombre de une, et audit dispositif d'acquisition d'images (14), au moins au nombre de un.

10. Système tel que revendiqué dans la revendication 9, dans lequel lesdits moyens de blocage/immobilisation (24) de l'échantillon (S) comprennent au moins une paire d'éléments de serrage opposés (24a, 24b) adaptés pour serrer l'échantillon (S) entre eux.

11. Système tel que revendiqué dans la revendication 10, dans lequel ladite paire d'éléments de serrage (24a, 24b), au moins au nombre de une, comprend une paire de rouleaux ou cylindres inférieurs (24a) et une paire de rouleaux ou cylindres supérieurs (24b) adaptées pour venir en contact l'une avec l'autre dans un état de serrage de l'échantillon (S).

12. Système tel que revendiqué dans l'une quelconque des revendications 10 ou 11, dans lequel les surfaces latérales desdits éléments de serrage (24a, 24b) présentent des rainures et des reliefs alternés qui, dans l'état de serrage de l'échantillon (S), entrent en prise de manière complémentaire, de manière à contribuer à une répartition transversalement homogène de l'échantillon (S) pendant le serrage.

13. Système tel que revendiqué dans l'une quelconque des revendications 10 à 12, dans lequel ladite paire d'éléments de serrage (24a, 24b), au moins au nombre de une, présente des surfaces de contact avec l'échantillon (S) présentant un revêtement d'un matériau adapté pour assurer un frottement s'opposant au glissement de l'échantillon (S).

14. Système tel que revendiqué dans l'une quelconque des revendications précédentes, comprenant en outre des moyens (26) permettant de tendre l'échantillon (S), dans lequel lesdits moyens de tension d'échantillon (26) comprennent de préférence au moins un rouleau adapté pour tourner autour de son propre axe, dont la surface latérale est recouverte d'un matériau adapté pour assurer un frottement s'opposant au glissement de l'échantillon (S).
